# EUROPÄISCHE PATENTSCHRIFT

(11) **EP 1 274 471 B1**
(45) Veröffentlichungstag und Bekanntmachung des Hinweises auf die Patenterteilung: **03.01.2007**
(21) Anmeldenummer: 01937984.1
(22) Anmeldetag: 10.04.2001
(51) Int. Cl.: A61L 27/34, A61L 33/06, C08G 79/02

(54) **Verwendung von Folien aus Poly-Tri-Fluor-Ethoxypolyphosphazenen zur Umhüllung von medizinischen Vorrichtungen**
Use of Poly-tri-fluoro-ethoxypolyphosphazene for covering medical devices
Utilisation de films en poly-tri-fluoro-ethoxypolyhosphazene pour envelopper des objets médicaux

(30) Priorität: 11.04.2000 US 196083 P; 22.04.2000 DE 10019982
(43) Veröffentlichungstag der Anmeldung: 15.01.2003
(73) Patentinhaber: Polyzenix GmbH, 89077 Ulm (DE)
(72) Erfinder: GRUNZE, Michael, 69151 Neckargemünd (DE); GRIES, Claudia, 89075 Ulm (DE)
(74) Vertreter: Teipel, Stephan
(86) Internationale Anmeldenummer: PCT/DE2001/001398
(87) Internationale Veröffentlichungsnummer: WO 2001/080919

(56) Entgegenhaltungen:
- WO-A-01/70296
- WO-A-99/16477
- DE-A- 19 613 048
- US-A- 5 548 060
- ALLCOCK: "POLYPHOSPHAZENES" , INORGANIC POLYMERS, 1992, PAGES 61-139 XP000866367 Seite 95 -Seite 117

## Beschreibung

Die vorliegende Erfindung betrifft die Verwendung von Folien aus einem antithrombogenen und körperverträglichen Polymer Umhüllung von medizinischen Vorrichtungen, wie beispielsweise Stents.

Die größten Komplikationen, die durch künstliche Implantate auftreten, sind zum einen in der vermehrten Thrombozytenablagerung auf der körperfremden Oberfläche zu sehen. Zum anderen spielt das Verhalten gegenüber Bakterien, Makrophagen und Proteinen, die sich auf den Oberflächen der Implantate ablagern, eine zentrale Rolle, da diese Ablagerungen wesentlich zu Entzündungen und anderen Problemen beim Einwachsen der Implantate führen.

Eines der Probleme, die auftreten können, ist beispielsweise die verstärkte Zellproliferation und die Entzündung von verletztem Gewebe, das mit dem künstlichen Implantat in Berührung kommt. Beispielsweise treten bei Gefäßimplantaten, z.B. sogenannten Stents, neben den bereits bekannten Problemen der verstärkten Thrombenbildung Restenosen (d.h. die Wiederverengung des Blutgefäßes im durch eine Angioplastie aufgedehnten Bereich, häufig der Stentbereich) auf. Diese Komplikationen werden unter anderem infolge der Aktivierung des Gerinnungs- und Immunsystems durch den implantierten Fremdkörper sowie eine Schädigung der Gefäßwand bei der Stentimplantation im Verlauf einer Angioplastie ausgelöst. Die Konsequenz ist die sogenannte Restenose (Wiederverschluß der Ader) sowie mögliche Entzündungen im behandelten Bereich, die einer unverzüglichen medikamentösen und oft auch einer chirurgischen Behandlung bedürfen.

Eine Möglichkeit, mit der versucht wird, diese Komplikationen, die durch eine verstärkte Zellproliferation im Bereich des Stents entstehen, zu verhindern, ist, die Verwendung von umhüllten, sogenannten gecoverten Stents. Aus dem Stand der Technik sind eine Fülle von Materialien und auch umhüllte Stents bekannt und untersucht, die für die Herstellung von solchen Hüllen Verwendung finden. So wird beispielsweise in der WO 9856312 eine expandierbare Hülle aus e-PTFE für diesen Zweck verwendet. Andere Materialien für diese Verwendung sind in der EP 0810845 zitiert und nennen beispielsweise Polymere, die in den Schriften US 4,883,699 und US 4,911,691 zitiert sind. Weitere Polymere, die für den genannten Zweck benannt sind, sind z.B. hydrolysiertes Polyacrylnitril (US 4,480,642) hydrophile Polyether (US 4,798,876) und Polyurethane-di-Acrylate (US 4,424,395), weiterhin sind verschiedene Hydrogele bekannt, die für diesen Zweck eingesetzt werden können. Die Reihe der potentiell anwendbaren Materialien kann weiterhin ergänzt werden durch Poly-Vinyl-Pyrrolidone (PVP) - Polymere, Poly-Vinyl-Alkohole (PVA), p(Polyethylenoxid)-Polymere (PEO) und Poly-Hydroxy-Ethyl-Methacrylate p(HEMA). Weiterhin erwähnen Schriften die Anwendung einer Reihe von Standardmaterialien wie Polyurethane, Polyethylene und Polypropylene als mögliche Werkstoffe. Ebenso sind Mischungen dieser Werkstoffe untereinander bekannt. Eine Reihe weiterer Materialien sind aus der EP 0804909 bekannt.

Die Eigenschaften dieser Verbindungen sind unterschiedlich und es kann davon ausgegangen werden, daß jeder dieser Werkstoffe besondere Eigenschaften für bestimmte Anwendungen aufweist. So ist z. B. PVA sehr gut in Flüssigkeiten löslich. Andere Materialien weisen eine gute Blutverträglichkeit auf. Wiederum andere Materialien sind besonders gut streckbar. Jedoch weisen bedauerlicherweise alle Materialien Defizite in bestimmten Bereichen auf. PVA zeigt zum Beispiel keine besonders gute Blutverträglichkeit. ε-PTFE ist beispielsweise sehr gut streckbar und weist auch eine gute Blutverträglichkeit auf, jedoch ist dieses Material ausgesprochen schwierig zu handhaben und die Herstellung solcher Hüllen erfordert eine Reihe von Verarbeitungsschritten (WO 96/00103). Bei anderen Materialien können elastische Eigenschaften nur durch die Zugabe von Weichmachern erreicht werden, welche die Blut- und Körperverträglichkeit vermindern und durch Ausschwemmen der "Weichmacher" zusätzlich eine Belastung für den Patienten darstellen.

Das bedeutet unter anderem, daß gegenwärtig Patienten aufgrund der unzureichenden Eigenschaften der vorhandenen Werkstoffe während der postoperativen Behandlung nach einer Angioplastie unter anderem Gerinnungshemmer erhalten müssen (Vitamin K Antagonisten), deren Dosierung jedoch problematisch ist.

Ebenso bedeutet es, daß die Restenosehäufigkeit bei handelsüblichen Stents bei ca. 30-50% innerhalb von 6 Monaten nach erfolgter Angioplastie liegt.

Diese Rate soll durch die Verwendung von umhüllten Stents gesenkt werden, indem verhindert wird, daß Zellgewebe in den Aderraum wachsen kann. Jedoch stößt diese Technik auf Grenzen, die vor allem durch die Materialien und deren physikalisch-chemische Eigenschaften und die Oberflächenbeschaffenheit dieser Materialien gezogen werden.

Die polymere Verbindung Poly[bis(trifluoroethoxy)phosphazen] zeigt als Volumenmaterial eine gute antithrombogene Wirkung (vgl. Tur, Untersuchungen zur Thrombenresistenz von Poly[bis(trifluoroethoxy)phosphazen] und Hollemann Wiberg, "Stickstoffverbindungen des Phosphors", Lehrbuch der anorganischen Chemie, 666-669, 91.-100. Auflage, Walter de Gruyter Verlag, 1985, sowie Tur, Vinogradova, u.a. "Entwicklungstendenzen bei polymeranalogen Umsetzungen von Polyphosphazen", Acta Polymerica 39, 424-429, Nr. 8, 1988). Weiterhin wurde Polyphosphazene in der Patentschrift DE 196 13 048 als Beschichtung für die Beschichtung künstlicher Implantate eingesetzt.

Jedoch kann diese Substanz alleine das Zellwachstum, welches zu Restenosen führt, nicht begrenzen oder verringern.

WO 01/70296 A1 beschreibt Polyphosphazenderivate und ihre Verwendung, die biokompatible Eigenschaften aufweisen und die einem Überzug eines Gegenstands wie einem medizinischen Gerät bakterielle Resistenz verleihen. WO 99/16477 betrifft ein radioaktiv markiertes, antithrombogenes Polymer und dessen Verwendung als Bestandteil therapeutischer Vorrichtungen zur Verhinderung von übermäßiger Zellproliferation oder Narbenbildung sowie Vorrichtungen, welche das radioaktiv markierte, antithrombogene Polymer umfassen, wie beispielsweise Pflaster oder künstliche Implantate mit einem biokompatiblen Überzug. DE-A-196 13 048 A beschreibt künstliche Implantate mit einem biokompatiblen Überzug, der mindestens eine Verbindung mit antithrombogenen Eigenschaften, vorzugsweise Poly[bis(trifluorethoxy)phosphazen], enthält, sowie Verfahren zu deren Herstellung. Allcock ("Polyphosphazenes", Inorganic Polymers, 1992, Seiten 95 ff.) beschreibt Polyphosphazene im allgemeinen, ihre Entwicklung, Synthese, Eigenschaften und ihre Verwendung als beispielsweise biomedizinische Materialien. Das US-Patent 5,548,060 beschreibt ein Verfahren zur Alkylsulfonierung von polymeren und zyklischen trimeren Phosphazenen, welches die Reaktion des Phosphazens mit einem Sulfon beinhaltet. Das Verfahren kann zur Herstellung sulfonierter Phosphazene verwendet werden, die als biomedizinische Materialien, Membranen, reversibel quervernetzbare Polymere, grenzflächenaktive Mittel und Polyelektrolyte einsetzbar sind.

Somit liegt der vorliegenden Erfindung die Aufgabe zugrunde, eine Umhüllung für medizinische Vorrichtungen, wie beispielsweise Katheter oder Stents aller Art, zur Verfügung zu stellen, welche einerseits ausgezeichnete mechanische und körperverträgliche Eigenschaften aufweisen soll, um dadurch die Biokompatibilität umhüllter medizinischer Vorrichtungen zu verbessern, andererseits sollen auch die vorgenannten Folgeschäden nach erfolgter Behandlung bzw. Implantation verhindert oder verringert werden. Insbesondere soll durch die Bereitstellung der Umhüllung unkontrolliertes Zellwachstum, welches beispielsweise nach Stentimplantation zu Restenosen führt, verhindert bzw. verringert werden. Weiterhin soll zusätzlich noch eine Verringerung der entzündlichen Reaktionen erreicht werden, die durch die Einbringung eines Fremdkörpers in den Organismus eine übliche Reaktion ist und die Gabe von Antibiotika erfordert.

Diese Aufgabe wird durch die Verwendung einer Folie aus einem biokompatiblen Polymer mit der folgenden allgemeinen Formel (I) 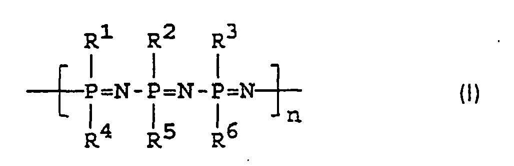 wobei n für 2 bis ∞ steht, R¹ bis R⁶ gleich oder unterschiedlich sind und einen Alkoxy-, Alkylsulfonyl-, Dialkylamino- oder Aryloxyrest oder einen Heterocycloalkyl- oder Heteroarylrest mit Stickstoff als Heteroatom bedeuten, zur Umhüllung von medizinischen Vorrichtungen, gelöst.

Der Polymerisationsgrad des für die Herstellung der Folie und der hieraus gefertigten Hülle verwendeten Polymers gemäß Formel (I) soll 2 bis ∞ betragen. Bevorzugt ist jedoch ein Bereich für den Polymerisationsgrad von 20 bis 200000, mehr bevorzugt von 40 bis 10.000.000.

Weiterhin soll das für die Herstellung verwendete Polymer folgenden Anforderungen gehorchen:

Vorzugsweise ist mindestens einer der Reste R¹ bis R⁶ im verwendeten Polymer ein Alkoxyrest, der mit mindestens einem Fluoratom substituiert ist.

Die Alkylreste in den Alkoxy-, Alkylsulfonyl- und Dialkylaminoresten sind beispielsweise gerad- oder verzweigtkettige Alkylreste mit 1 bis 20 Kohlenstoffatomen, wobei die Alkylreste beispielsweise mit mindestens einem Halogenatom, wie ein Fluoratom, substituiert sein können.

Beispiele für Alkoxyreste sind Methoxy-, Ethoxy-, Propoxy- und Butoxygruppen, die vorzugsweise mit mindestens einem Fluoratom substituiert sein können. Besonders bevorzugt ist die 2,2,2-Trifluoroethoxygruppe. Beispiele für Alkylsulfonylreste sind Methyl-, Ethyl-, Propyl- und Butylsulfonylgruppen. Beispiele für Dialkylaminoreste sind Dimethyl-, Diethyl-, Dipropyl- und Dibutylaminogruppen.

Der Arylrest im Aryloxyrest ist beispielsweise eine Verbindung mit einem oder mehreren aromatischen Ringsystemen, wobei der Arylrest beispielsweise mit mindestens einem, vorstehend definierten Alkylrest substituiert sein kann. Beispiele für Aryloxyreste sind Phenoxy- und Naphthoxygruppen und Derivate davon.

Der Heterocycloalkylrest ist beispielsweise ein 3- bis 7- Atome enthaltendes Ringsystem, wobei mindestens ein Ringatom ein Stickstoffatom ist. Der Heterocycloalkylrest kann beispielsweise mit mindestens einem, vorstehend definierten Alkylrest substituiert sein. Beispiele für Heterocycloalkylreste sind Piperidinyl-, Piperazinyl-, Pyrrolidinyl- und Morpholinylgruppen und Derivate davon. Der Heteroarylrest ist beispielsweise eine Verbindung mit einem oder mehreren aromatischen Ringsystemen, wobei mindestens ein Ringatom ein Stickstoffatom ist. Der Heteroarylrest kann beispielsweise mit mindestens einem, vorstehend definierten Alkylrest substituiert sein. Beispiele für Heteroarylreste sind Pyrrolyl-, Pyridinyl-, Pyridinolyl-, Isochinolinyl- und Chinolinylgruppen, und Derivate davon.

In einer bevorzugten Ausführungsform der vorliegenden Erfindung besteht die Folie und die hieraus gefertigte Hülle aus dem Polymer, das ein mit ³²P-, ³³P- oder As- oder Sb - Isotopen markiertes Poly[bis(trifluoroethoxy)phosphazen] ist.

Künstliche Implantate, Pflaster, künstlichen Blutgefäße, Stents, Katheter, Urether oder sonstigen Implantate ohne direkten Blutkontakt können von der Folie aus einem biokompatiblen Polymer umhüllt sein.

Die gefertigte Umhüllung kann in Verbindung mit einer Beschichtung oder ohne eine besondere Vorbehandlung des Implantats verwendet werden. Weiterhin können die erfindungsgemäß umhüllten Stents nicht nur in arteriellen Gefäßen, sondern auch in venösen Gefäßen, im Magen-Darm-Trakt, in der Speise- oder Luftröhre oder den Harnwegen verwendet werden.

Der biokompatible Überzug eines künstlichen Implantats weist beispielsweise eine Dicke von etwa 1 nm bis etwa 100 µm, vorzugsweise bis etwa 10 µm und besonders bevorzugt bis etwa 1 µm auf.

Das als Substrat verwendete Implantatmaterial weist keine besondere Beschränkung auf und kann jedes Implantatmaterial, wie Kunststoffe, Metalle, Metallegierungen und Keramiken, sein. Insbesondere kann das Implantatmaterial ein keramisches oder ein metallisches Stentmaterial sein.

Die Herstellung der Folie und der Hülle erfolgt im allgemeinen durch:

Eine Lösung, die mindestens eine Verbindung der allgemeinen Formel 1 in einer Konzentration, von 0,1 % -99% enthält, in einem Lösungsmittel, wobei dieses Lösungsmittel organisch und polar ist. Beispielsweise können hier Ethylacetat, Aceton, THF, Toluol oder Xylole verwendet werden. Ebenso sind auch Mischungen dieser Lösungsmittel verwendbar oder können durch andere Lösungsmittel ergänzt werden. Diese Lösung wird auf ein Substrat gegeben, das keine oder eine geringe Adhäsion gegenüber dem Polymer zeigt, wie z.B. Glas, Silizium, verschiedene Keramiken oder andere entsprechende Werkstoffe, wie Polymere (PDMS, Teflon, PMMA, Polycarbonate oder Silicone). Die Oberflächen der aufgeführten Substrate können auch chemisch modifiziert sein, z.B. durch Einführung bestimmter funktioneller Gruppen (-NH2, -OH, -COOH, -COH, - COOMe, -CF3 usw.).

Das Verdampfen des Lösungsmittels kann ohne weitere Maßnahmen ablaufen, jedoch im besten Fall wird die Konzentration des Lösungsmitteldampfes über dem Substrat kontrolliert eingestellt, wie auch der Druck und die Temperatur. Zu Beginn der ersten Trocknungsphase soll die Atmosphäre über dem beschichteten Substrat mit Lösungsmitteldampf gesättigt sein, wobei die Konzentration des Lösungsmitteldampfes dann über mehrere Stunden langsam reduziert wird. Die Temperatur kann von -30°C bis + 90 °C variieren. Der Druck kann während der ersten Trocknungsphase eine Rampe von Normaldruck bis Wasserstrahlvakuum (20 Torr) durchlaufen. Nach der ersten Trocknungsphase wird das beschichtete Substrat für eine bestimmte Zeit im Ölpumpenvakuum (0,1 Torr) weiter getrocknet.

Das auf dem Substrat getrocknete Polymer der Verbindung I kann dann als Folie vom Substrat abgezogen werden. Je nach Konzentration der Polymerlösung von Verbindung I und den angesprochenen Bedingungen während der ersten Trocknungsphase erhält man Folien verschiedener Schichtdicken von 0,1 µm bis 300 µm oder dicker, vorzugsweise im Bereich von 0,5 µm bis 30 µm, und besonders bevorzugt um 5 µm.

In einer besonderen Ausführungsform können die Folie oder Hülle gemäß den aufgeführten Arbeitsschritten auch mikrostrukturiert hergestellt werden.

In diesem Fall ist das Substrat, auf das die Lösung der Verbindung I aufgegeben wird, mikrostrukturiert. Die Struktur des Substrates wird auf die Struktur der Folie des verwendeten Polymers 1:1 übertragen. Bei den Strukturgrößen des Substrates ist man nicht festgelegt. So können Strukturen in der Größenordnung von Nanometern, Mikrometern oder noch größer oder kleiner hergestellt werden. Weiterhin unterliegt man in der Ausführungsform der Strukturierung keiner Einschränkung. So können alle Strukturen hergestellt und verwendet werden, die photolithographisch oder mit Elektronenstrahl oder mit Ionenstrahl oder mit dem Laser oder mittels anderer Techniken erzeugt werden können. Insbesondere können Strukturen erzeugt werden, die ein besonders strömungsgünstiges Profil aufweisen. Wie beispielsweise Lotosstrukturen oder Strukturen ähnlich der aus dem Flugzeugbau bekannten "Haifischhaut". Der besondere Vorteil dieser Strukturen und deren Verwendung bei der Herstellung von Folien und Hüllen liegt in der Verringerung der sogenannten Kontaktaktivierung des Gerinnungssystems.

Das auf dem Substrat getrocknete Polymer der Verbindung I kann dann als strukturierte Folie vom Substrat abgezogen und weiter verarbeitet werden. Je nach Konzentration der Polymerlösung von Verbindung I und den angesprochenen Bedingungen während der ersten Trocknungsphase erhält man Folien verschiedener Schichtdicken von 0,1 *µ*m bis 300 *µ*m oder dicker, vorzugsweise im Bereich von 0,5 *µ*m bis 30 *µ*m, und besonders bevorzugt um 5 *µ*m.

Die Mikrostrukturierung der Folie kann auch durch ein direktes "Schreiben" auf der bereits vorliegenden Folie selbst mittels Laser-, Elektronen- oder Röntgenstrahlen erhalten werden oder aber durch "schmelzstrukturieren", wobei ein dünner Draht auf die Schmelztemperatur des Polymers gebracht wird, der dann die gewünschte Struktur durch direkten Kontakt in die Folie einschmilzt.

Besondere Vorteile können durch diese Strukturierung dadurch erreicht werden, daß Strukturen in die Folie eingeprägt werden, die das Strömungsverhalten von Flüssigkeiten besonders günstig gestalten (z. B. Haifischhaut oder Lotoseffekt).

Dadurch kann im Fall von umhüllten Stents die Kontaktaktivierung von Blut weiter gemindert und die Gefahr der Thrombozytenaggregation weiter gesenkt werden. Diese Art der Strukturierung ist jedoch nicht nur auf die Herstellung von umhüllten Stents beschränkt, sondern kann auch zur Herstellung von Kathetern oder Schläuchen in durchflossenen Systemen, wie Supportsystemen, Angioplastiekathetern, Urether etc. verwandt werden. Ebenso besteht die Möglichkeit auf diese Art beispielsweise das Innenleben von Dialysegeräten zu gestalten und dadurch den Bedarf an Heparin zu senken.

Die Hülle wird gemäß dem nachfolgenden Vorgehen hergestellt.

Die nach dem vorhergehenden Verfahren erhaltene Folie der Verbindung I wird auf die Größe der Stents zugeschnitten (+2 mm). Die Stents werden auf eine Halterung aufgebracht und mit der Folie der Verbindung I so umwickelt, daß die Folie an beiden Enden gleichmäßig 1 mm übersteht. Die Umwicklung der Stents mit Folie kann eine Lage oder auch mehrere Lagen dick sein. Die Enden der so entstandenen Hülle sowie die einzelnen Lagen der Folie werden dann im heißen Lösungsmitteldampf von allen Seiten, innen und außen, "verschweißt". Hierzu wird das Lösungsmittel auf eine Temperatur von 40-120°C erhitzt, im besten Fall auf 80°C oder auf eine höhere Temperatur. Der Lösungsmitteldampf steigt auf und strömt aus einer sehr feinen Röhre, die mit einer oder mehreren Düsen versehen ist. Diese Röhre kann je nach Länge und Material der Röhre beheizt sein und ist in einem bestimmten Winkel zum aufsteigenden Lösungsmitteldampf angebracht, so daß der in der Düsenröhre zum Teil kondensierende Lösungsmitteldampf wieder zurück ins Vorratsgefäß ablaufen kann, ohne die Düsen zu verstopfen oder den aufsteigenden Lösungsmitteldampf am Ausströmen zu hindern.

Die ebenfalls durch die Verwendung der Folie zur Umhüllung erhaltenen Implantate behalten überraschenderweise die ausgezeichneten mechanischen Eigenschaften des Vorrichtungs- bzw. Implantatmaterials bei. Dadurch wird nicht nur die Biokompatibilität derartiger künstlicher Implantate drastisch verbessert, sondern es wird auch unkontrolliertes Zellwachstum, welches beispielsweise nach einer Stentimplantation zu Restenosen führt, infolge der Verhinderung des Zellwachstums in den Aderraum verringert. Weiterhin kann durch die Verwendung einer erfindungsgemäß mikrostrukturierten Folie die Kontaktaktivierung des Gerinnungssystems praktisch unterbunden werden.

## Patentansprüche

1. Verwendung einer Folie aus einem biokompatiblen Polymer mit der folgenden allgemeinen Formel (I) 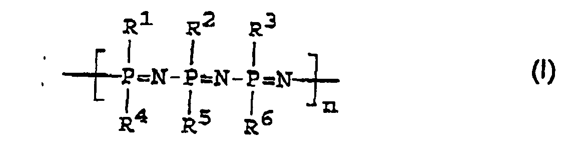 wobei n für 2 bis = steht, R¹ bis R⁶ gleich oder unterschiedlich sind und einen Alkoxy-, Alkylsulfonyl-, Dialkylamino- oder Aryloxyrest oder einen Heterocycloalkyl- oder Heteroarylrest mit Stickstoffheteroatom bedeuten, zur Umhüllung von medizinischen Vorrichtungen.

2. Verwendung nach Anspruch 1, wobei die medizinische Vorrichtung aus künstlichen Implantaten, Pflastern, künstlichen Blutgefäßen, Stents, Kathetern, Urethern oder sonstigen Implantaten ohne direkten Blutkontakt ausgewählt ist.

3. Verwendung nach Anspruch 1 oder 2, wobei die Folie eine mikro-strukturierte Folie ist, erhältlich durch Aufbringen des biokompatiblen Polymers in Lösung auf einen mikrostrukturierten Stempel, Verdampfen des Lösungsmittels und Abziehen der mikrostrukturierten Folie.

4. Verwendung nach Anspruch 3, wobei die Strukturen der mikrostrukturierten Folie regelmäßig sind.

5. Verwendung nach Anspruch 4, wobei die Oberfläche der mikrostrukturierten Folie strömungsgünstig ist.

6. Verwendung nach Anspruch 1 oder 2, wobei die Folie eine mikro-strukturierte Folie ist, worin die Struktur direkt mittels Laser-, Elektronen- oder Röntgenstrahlung oder einem heißen Draht auf der Folie erzeugt wird.

## Claims

1. Use of a film made of a biocompatible polymer having the following general formula (I) 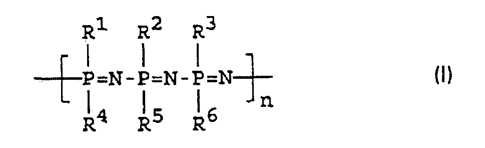 wherein n is 2 to ∞, R¹ to R⁶ are equal or different and represent an alkoxy, alkylsulfonyl, dialkylamino or aryloxy residue or a heterocycloalkyl or heteroaryl residue with nitrogen heteroatom, for sheathing medical devices.

2. Use according claim 1, wherein the medical device is selected from artificial implants, patches, artificial blood vessels, stents, catheters, ureters or other implants without direct blood contact.

3. Use according to claim 1 or 2, wherein the film is a microstructured film obtainable by applying the biocompatible polymer in solution to a microstructured stamp, evaporating the solvent and peeling off the microstructured film.

4. Use according to claim 3, wherein the structures of the microstructured film are regular.

5. Use according to claim 4, wherein the surface of the microstructured film has favorable flow characteristics.

6. Use according to claim 1 or 2, wherein the film is a microstructured film, the structure of which is produced directly on the film by means of laser, electron or X-ray radiation or a hot wire.

## Revendications

1. Utilisation d'une feuille de polymère biocompatible de formule générale (I) suivante : 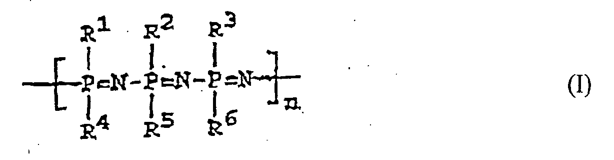 dans laquelle n est compris entre 2 et ∞, R¹ et R⁶ sont identiques ou différents et sont un radical alcoxy, alkylsulfonyl, dialkyl amino ou aryloxy ou un radical hétérocycloalkyl ou hétéroaryl avec un hétéro-atome azote, pour envelopper des dispositifs médicaux.

2. Utilisation selon la revendication 1, dans laquelle le dispositif médical est choisi parmi les implants artificiels, les emplâtres, les vaisseaux sanguins artificiels, les endoprothèses vasculaires (stent), les cathéters, des urethères ou d'autres implants sans contact direct avec le sang.

3. Utilisation selon la revendication 1 ou 2, dans laquelle le feuille est une feuille de microstructure, disponible grâce à la capture des polymères biocompatibles en solution dans un tampon de microstructure, à l'évaporation du solvant et au retrait de la feuille de microstructure.

4. Utilisation selon la revendication 3, dans laquelle les structures de la feuille de microstructure sont régulières.

5. Utilisation selon la revendication 4, dans laquelle la surface de la feuille de microstructure est propice à l'écoulement.

6. Utilisation selon la revendication 1 ou 2, dans laquelle la feuille est une feuille à microstructure, dans laquelle la structure sera générée directement au moyen de rayonnement laser, à électrons ou X ou d'un fil brûlant sur la feuille.
